# FASCICULE DE BREVET EUROPEEN

(11) **EP 1 413 329 B1**
(45) Date de publication et mention de la délivrance du brevet: **14.12.2005**
(21) Numéro de dépôt: 03292670.1
(22) Date de dépôt: 24.10.2003
(51) Int. Cl.: A61M 39/02

(54) **Chambre implantable par voie sous-cutanée**
Implantierbare Kammer
Implantable chamber

(30) Priorité: 25.10.2002 FR 0213386
(43) Date de publication de la demande: 28.04.2004
(73) Titulaire: B. Braun Medical SAS, 92100 Boulogne Billancourt (FR)
(72) Inventeur: Bunodiere, Michel, 92200 Neuilly sur Seine (FR); Nadal, Guy, 86000 Poitiers (FR)
(74) Mandataire: Arnaud, Jean

(56) Documents cités:
- EP-B- 0 678 302
- WO-A-02/083208
- DE-A- 19 745 654
- US-A- 5 328 465

## Description

L'invention concerne une chambre implantable d'infusion d'un médicament, destinée à être implantée en sous-cutané par une incision dans un corps.

On connaît déjà de telles chambres qui comprennent un réservoir de médicament, une zone d'accès au réservoir, accessible depuis l'extérieur du corps et destinée à permettre le remplissage du réservoir de médicament, et un conduit de diffusion relié au réservoir et s'étendant à l'extérieur de celui-ci.

De telles chambres peuvent aussi être utilisées en sens inverse, c'est-à-dire pour le prélèvement d'un liquide corporel (sang en particulier), le liquide se rassemblant dans la chambre à partir de laquelle il peur être prélevé. De telles chambres entrent aussi dans le cadre de l'invention, mais on décrit celle-ci dans la suite uniquement en référence à une chambre d'infusion d'un médicament, sans limiter la portée de l'invention aux seules chambres d'infusion.

La chambre est destinée à être implantée sous la peau d'un patient. Au cours d'une telle opération, le chirurgien incise le derme, par exemple au niveau du thorax, et insère une chambre implantable entièrement sous la peau. Pendant l'opération, l'extrémité d'un conduit de sortie de la chambre est accessible au niveau de l'incision pour la raccordement d'un cathéter qui rejoint l'emplacement d'infusion, par exemple la veine jugulaire.

Après implantation, une seringue, introduite par la zone d'accès, peut être utilisée comme source de médicament devant être diffusé progressivement dans le corps, ou pour un prélèvement.

Le document US-A-5 951 512 décrit une chambre implantable destinée à transférer par voie transdermique un liquide entre un réservoir et une zone de diffusion à l'intérieur du corps d'un patient. Elle comporte un réservoir circulaire de médicament, une joue de support entourant le bas du réservoir, une zone d'accès au réservoir, située vers le sommet du réservoir, accessible depuis l'extérieur du dispositif, et destinée à permettre le remplissage du réservoir de médicament, et un conduit de diffusion relié au réservoir, s'étendant radialement ou tangentiellement vers l'extérieur de celui-ci

Le document DE-197 45 654 décrit aussi une chambre implantable ayant un réservoir circulaire de médicament et une plaque de support entourant le réservoir, un conduit de diffusion relié au réservoir s'étendant radialement vers l'extérieur de celui-ci.

Les documents US-5 328 465, WO 02/083208, EP-678 302 et AT-391 813 décrivent des pompes de médicament, et non des chambres implantables. Elles comportent toutes un corps de section circulaire, de forme cylindrique ou lenticulaire, dont part un conduit de diffusion qui forme un coude tel que le conduit de diffusion a une direction sensiblement tangente au réservoir.

Ainsi, on connaît déjà une chambre implantable d'infusion d'un médicament, destinée à être implantée en sous-cutané par une incision dans un corps, qui comprend un réservoir de médicament, une zone d'accès au réservoir située à un sommet de celui-ci, accessible depuis l'extérieur du corps et destinée à permettre le remplissage du réservoir de médicament, et un conduit de diffusion relié au réservoir et s'étendant à l'extérieur de celui-ci, le conduit de diffusion ayant une portion terminale externe.

Une telle chambre pose les problèmes suivants. Comme sa mise en place nécessite une incision, elle est traumatisante, et il est souhaitable de réduire autant que possible ce traumatisme. La chambre constitue un corps étranger qui occasionne un certain inconfort qui dépend notamment de son volume et de sa forme, ainsi que de la robustesse de sa fixation sous la peau. Enfin, un autre facteur important est sa commodité de mise en place par le chirurgien, car plus l'intervention est longue et plus le traumatisme est important.

L'invention permet la solution de ces problèmes par la combinaison d'une forme profilée qui rend difficile la rotation de la chambre sur elle-même, avec un conduit de sortie ayant une orientation telle que le raccordement du cathéter au conduit s'effectue facilement et rapidement tout en réduisant le traumatisme.

Plus précisément, selon l'invention ces résultats sont obtenues grâce aux caractéristiques de la revendication 1.

De préférence, l'enveloppe a aussi une forme effilée vers le sommet du triangle, dans la direction de l'épaisseur, c'est-à-dire perpendiculairement à la paroi de base.

Ainsi, on tire avantage de la synergie entre la forme extérieure effilée qui favorise la mise en place sous-cutanée dans un logement aussi réduit que possible sous la peau, et une sortie du conduit de diffusion orientée dans la direction de l'incision, qui permet un raccordement d'un cathéter avec un traumatisme minimal.

Le conduit de diffusion est situé dans une zone de la chambre opposée à l'extrémité effilée de l'enveloppe, de sorte que cette partie effilée pénètre au fond du logement sous-cutané, et le conduit de diffusion se trouve alors à proximité immédiate de l'incision faite par le praticien dans la peau du patient. Le raccordement de la partie terminale du conduit de diffusion au cathéter destiné à injecter le produit plus loin dans le corps est facile et rapide, et le praticien peut également fermer facilement l'incision, une fois la chambre en place sous la peau.

De préférence, le réservoir a une section sensiblement circulaire en coupe parallèle à la paroi de base de l'enveloppe.

Dans un mode de réalisation, la partie du conduit de diffusion la plus proche du réservoir est radiale par rapport à la paroi circulaire du réservoir, si bien que le conduit de diffusion comporte un coude entre sa partie la plus proche du réservoir et sa partie terminale. De préférence, le coude du conduit de diffusion est à l'intérieur de l'enveloppe. Il est alors avantageux que le coude forme un angle compris entre environ 100 et 150°.

Ces caractéristiques simplifient les conditions de fabrication de la chambre. Par raison de réduction du volume de la chambre, et pour que la ligne d'incision cutanée et la direction de la portion terminale du conduit de diffusion soient sensiblement parallèles l'une à l'autre, le coude du conduit de diffusion est situé dans l'enveloppe, mais à l'extérieur du réservoir.

En outre, ces caractéristiques facilitent la réalisation de la chambre en réduisant les risques de fuite au raccordement entre les différentes parties.

Dans un autre mode de réalisation, la partie du conduit de diffusion la plus proche du réservoir est sensiblement tangente à la paroi circulaire du réservoir.

Un autre aspect de l'invention concerne l'encombrement de l'ensemble du dispositif, notamment à l'endroit de la sortie du conduit de diffusion.

Pour réduire l'encombrement de la chambre et augmenter ainsi le confort pour le patient, il est avantageux que l'enveloppe forme un dégagement à un coin de son contour triangulaire, et que la partie terminale du conduit de diffusion débouche de l'enveloppe à l'endroit du dégagement.

Grâce à sa forme triangulaire, la chambre implantable n'a pas tendance à tourner sur elle-même ni à migrer sous la peau. Cependant, certains praticiens souhaitent fixer la chambre par suture. Dans ce cas, la chambre est avantageusement telle que l'enveloppe a un orifice qui traverse la paroi de base à proximité du côté du triangle opposé au sommet de forme effilée, l'orifice étant destiné au passage d'un fil de suture.

D'autres caractéristiques et avantages de l'invention ressortiront mieux de la description qui suit d'exemples de réalisation, faite en référence aux dessins annexés sur lesquels :
- la figure 1 illustre l'implantation d'une chambre traditionnelle d'infusion de médicament, implantée en sous-cutané, sur le corps d'un patient, par une incision horizontale ;
- la figure 2 illustre l'implantation d'un dispositif selon l'invention, par une incision verticale ;
- la figure 3 est une vue en plan d'une chambre selon un premier mode de réalisation de l'invention ;
- la figure 4 est une vue en élévation latérale dans le sens de la flèche IV de la figure 3 ;
- la figure 5 est une coupe selon la ligne V-V de la figure 4 ; et
- les figures 6 et 7 représentent en plan deux autres variantes de réalisation.

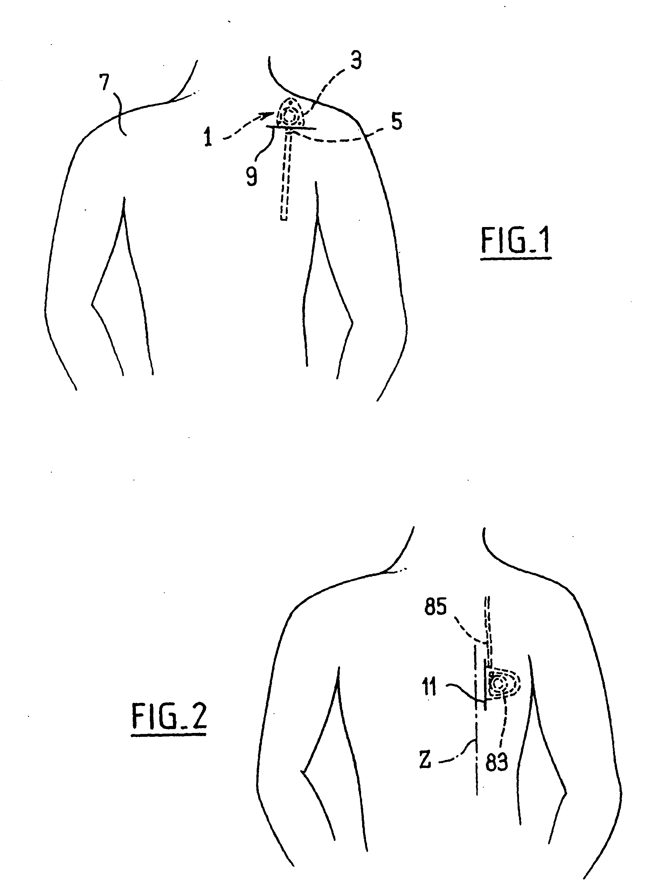

La figure 1 illustre l'implantation d'une chambre traditionnelle 1 d'infusion de médicament, implantée en sous-cutané, sur le corps 7 d'un patient, par une incision horizontale. La chambre comprend un corps 3 ayant un réservoir de médicament. Un conduit 5 de diffusion (ou de prélèvement) sort radialement du corps 3. Le conduit 5 est sensiblement perpendiculaire au corps à proximité de l'incision horizontale 9 qui a été réalisée au moins dans le derme du corps 7, pour implanter en sous-cutané la chambre.

La figure 2 illustre l'implantation d'une chambre d'infusion de médicament, implantée en sous-cutané, sur le corps d'un patient, par une incision 11 de direction verticale Z. La chambre comprend un conduit 85 de diffusion (ou de prélèvement) qui sort tangentiellement du corps. Le conduit 85 est sensiblement parallèle à l'incision 11 qui a été réalisée au moins dans le derme du corps, pour implanter en sous-cutané la chambre.

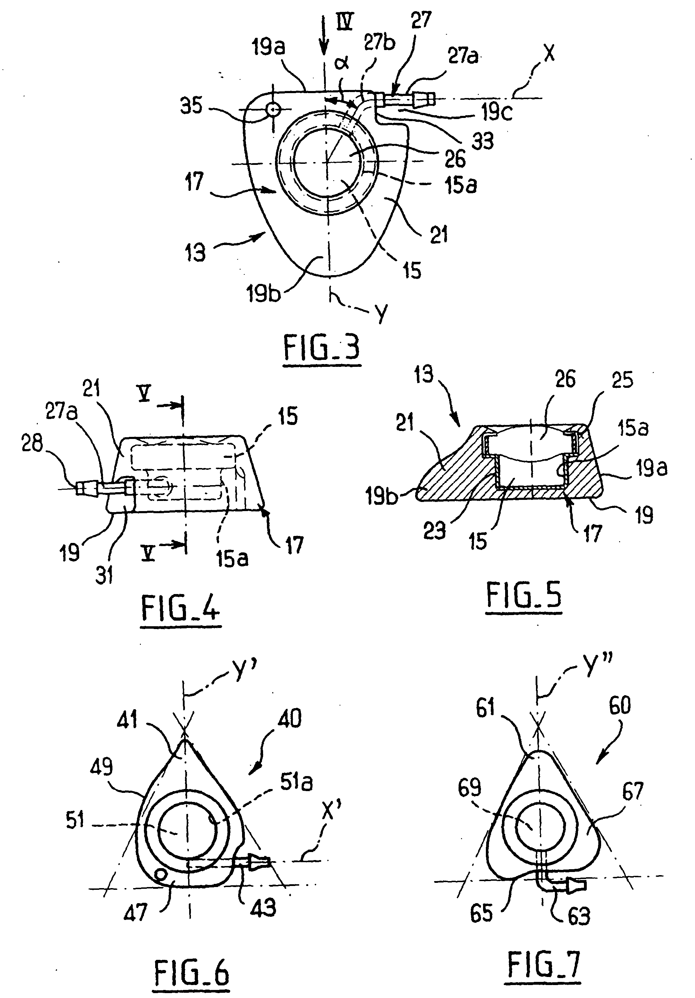

Sur les figures 3 à 5, une chambre implantable 13 selon l'invention comprend un réservoir 15 de liquide enrobé par une enveloppe extérieure moulée 17. L'enveloppe possède une paroi de base plane 19 pour l'appui de la chambre sur la chair du patient et une paroi latérale extérieure 21.

Le réservoir 15 est avantageusement métallique et comprend une coupelle inférieure 23 et une coiffe supérieure annulaire 25.

Le réservoir représenté a une section circulaire, en coupe parallèle à la paroi de base 19.

Au-dessus de la coupelle 23 est disposée une zone d'accès formée par une membrane 26 à la fois perforable et auto-obturable. Une coiffe annulaire 25 retient étroitement et étanche aux liquides la membrane 26 entre elle et la coupelle.

Un conduit métallique de diffusion 27 communique avec le réservoir. Il traverse radialement la paroi circulaire 15a du réservoir, puis passe dans l'enveloppe 17.

Dans le mode de réalisation considéré, le conduit 27 s'étend dans un plan horizontal parallèle à la paroi de base 19.

La base de l'enveloppe 17 représentée sur les figures 3 à 5 est sensiblement triangulaire, avec un côté frontal 19a et un sommet ou coin arrière 19b en direction duquel la paroi latérale est effilée, dans la partie de la chambre destinée à pénétrer le plus profondément dans le logement sous-cutané. On note en particulier sur la figure 5 que l'enveloppe 17 a aussi une forme effilée dans la direction de l'épaisseur, perpendiculairement à la paroi de base.

Dans la zone proche de l'incision, le conduit 27 s'étend à proximité immédiate du côté frontal 17a. Il traverse la paroi 21 de l'enveloppe près du coin avant droit 19c (haut à droite, sur la figure 3) et sa partie terminale 27a (qui s'étend à l'extérieur de l'enveloppe et se termine à l'extrémité libre 28) est dirigée sensiblement parallèlement au côté frontal de base 19a.

On peut également constater que la direction X de cette portion terminale 27a est transversale (ici perpendiculaire) à l'axe Y qui passe par le centre du côté frontal 19a et la pointe 19b, c'est-à-dire qui définit la direction d'effilement de l'enveloppe. Le conduit 27 est ainsi coudé. Sur les figures, il présente un unique coude 27b situé à l'intérieur de la zone de recouvrement de l'enveloppe, à l'extérieur du réservoir. Son raccordement au réservoir s'effectue avantageusement obliquement par rapport à l'axe Y, du côté du coin de sortie, pour diminuer l'angle et le nombre de coudes.

Le conduit délimite de préférence avec l'axe Y un angle α d'environ 10 à 60°, de préférence de 25° à 35°. Ainsi, le coude forme un angle compris entre 100 et 150°, de préférence entre 115 et 125°.

Pour limiter l'encombrement global, le coin de sortie du conduit présente un dégagement 31. Ainsi le conduit 27 traverse la paroi latérale 21 à l'endroit de l'une des faces 33 de ce dégagement.

Au coin opposé du côté frontal (en haut à gauche sur la figure 3), un orifice 35 permet au praticien de suturer (plus généralement lier) la chambre implantable sur la chair du patient. De préférence, la fixation de la chambre implantable au corps s'effectue par ce seul orifice, ou par plusieurs orifices le cas échéant. Cependant, grâce à la forme triangulaire de l'enveloppe qui permet une retenue robuste de la chambre, une telle suture est souvent inutile.

Bien entendu, le côté de sortie du conduit 27 peut indifféremment être "vers la droite" (comme sur la figure 3) ou vers la gauche (solution non illustrée).

Dans la variante de réalisation de la figure 6, la chambre implantable 40 a une forme de triangle dont les sommets du côté frontal sont très arrondis, donnant une forme sensiblement "en goutte d'eau". Cette réduction du côté frontal est avantageuse à certains emplacements d'implantation où l'incision doit être particulièrement courte.

La direction générale X' de sortie du conduit de diffusion 43 est transversale (en l'espèce perpendiculaire) à la direction générale Y' d'effilement qui passe par le sommet arrière 41 et par le milieu du côté avant 47 de la paroi de base 49 de cette chambre implantable.

Dans ce mode de réalisation, le conduit de diffusion 43 est sensiblement rectiligne (ou éventuellement très légèrement cintré) et se raccorde au réservoir 51 de façon sensiblement tangentielle à la paroi extérieure 51a de ce dernier. Ce mode de réalisation est moins avantageux que celui décrit en référence aux figures 3 à 5 parce que le raccord du conduit au réservoir est plus difficile à réaliser sans possibilité de fuite.

La figure 7 représente une autre variante ayant une forme effilée vers l'arrière (zone 61) et sensiblement triangulaire.

Dans ce cas, la chambre implantable 60 présente son conduit de raccordement 63 au centre du côté frontal 65. Ce conduit de raccordement est coudé à l'extérieur de l'enveloppe 67 et se raccorde au réservoir intérieur 69, radialement à sa paroi, sensiblement dans l'axe de la direction générale d'effilement Y" passant par la pointe arrière 61 et par le centre du côté frontal de base 71. Le coude est sensiblement à 90° par rapport à l'axe Y". Ce mode de réalisation est moins avantageux que celui des figures 3 à 5 parce que le coude n'est pas enrobé dans l'enveloppe et peut donc subir des forces de pivotement agissant directement sur sa fixation au réservoir.

La mise en place d'une chambre implantable selon l'invention peut s'effectuer comme suit et comme illustré sur la figure 2.

Tout d'abord, le praticien dispose d'une chambre implantable, effilée vers l'arrière et qui présente donc, vers son bord avant, une sortie latérale du conduit qui la raccorde au cathéter de diffusion.

Le praticien réalise alors une incision cutanée 11, par exemple dans la poitrine du patient, ou en zone sous-clavière.

L'incision est avantageusement inclinée par rapport à l'horizontale, et, en particulier, verticale comme indiqué sur la figure 2. Une telle orientation de l'incision permet dans certains cas de mieux la dissimuler, la cicatrice étant moins visible, et permet parfois une cicatrisation accélérée.

Au-delà de cette incision, le praticien ménage un logement 83 dans la chair du patient, juste sous la peau. Compte tenu de la forme effilée de la chambre implantable, il peut former légèrement en pointe le fond du logement 83.

Le praticien glisse alors la chambre implantable dans le logement, en ayant pris soin de choisir une chambre implantable dont l'orifice de sortie du conduit de raccordement est dirigé, suivant le cas, soit vers le haut du corps du patient, comme indiqué sur la figure 2, soit vers le bas (implantation sous-clavière).

Préalablement, le chirurgien a glissé le cathéter 85 dans le corps du patient, à l'aide d'un dispositif introducteur/élargisseur approprié. Ainsi, le cathéter a été glissé à l'intérieur d'un vaisseau, par exemple pour la distribution d'un médicament liquide anti-cancéreux.

Une extrémité du cathéter sort alors du corps du patient à l'endroit du logement sous-cutané 83 ou à proximité immédiate.

Le praticien met alors à longueur le cathéter et raccorde son extrémité sur l'embout du conduit de diffusion de la chambre implantable.

La portion terminale de ce conduit de diffusion, et la partie voisine du cathéter sont alors sensiblement parallèles à la direction Z de l'incision 11 et à proximité immédiate de celle-ci. Ceci facilite la manoeuvre par le praticien à la fois de la partie émergente du cathéter et de la chambre implantable.

Le praticien fixe ensuite la chambre implantable au corps du patient, par exemple par suture de l'orifice frontal (tel que 35 sur la figure 3). Un seul orifice de fixation à l'opposé du cathéter empêche le pivotement de la chambre implantable dans son logement 83.

Le chirurgien referme alors la peau à l'endroit de l'incision, par suture.

La chambre implantable équipée de son cathéter se trouve alors dans la position et à l'emplacement indiqués sur la figure 2, avec la zone d'accès juste sous la peau, donc accessible par une aiguille appropriée reliée à un réservoir de médicament. Le praticien peut aussi effectuer une ponction de sang par le réservoir de la chambre.

## Revendications

1. Chambre implantable d'infusion d'un médicament, destinée à être implantée en sous-cutané par une incision dans un corps, du type qui comprend :
- un réservoir (15, 51, 69) de médicament,
- une zone (26) d'accès au réservoir située à un sommet de celui-ci, accessible depuis l'extérieur du corps et destinée à permettre le remplissage du réservoir de médicament, et
- un conduit (27, 43, 63) de diffusion relié au réservoir et s'étendant à l'extérieur de celui-ci, le conduit de diffusion ayant ainsi une portion proche du réservoir et une portion externe (27a), et
- une enveloppe extérieure (17) entourant le réservoir et possédant une paroi de base (19, 49) et une paroi latérale extérieure (21), allant de la paroi de base vers le sommet du réservoir, le contour de la paroi de base (19, 49) étant sensiblement triangulaire et tel que l'enveloppe (17) a une forme effilée vers un sommet du triangle, et telle que :
- le conduit de diffusion est tel que sa partie la plus proche du réservoir est enrobée dans l'enveloppe, et sa portion externe est disposée à l'extérieur de l'enveloppe et s'étend en direction sensiblement parallèle au côté du triangle opposé au sommet de forme effilée **caractérisée en ce que** le conduit de diffusion est situé dans une zone de la chambre opposée à l'extrémité effilée de l'enveloppe.

2. Chambre selon la revendication 1, **caractérisée en ce que** l'enveloppe (17) a aussi une forme effilée vers le sommet du triangle dans la direction de l'épaisseur perpendiculaire à la paroi de base.

3. Chambre selon la revendication 2, **caractérisée en ce que** le réservoir (15, 51, 69) a une section sensiblement circulaire en direction parallèle à la paroi de base de l'enveloppe.

4. Chambre selon selon la revendication 2, **caractérisée en ce que** la partie du conduit de diffusion la plus proche du réservoir (15, 69) est radiale par rapport à la paroi circulaire du réservoir, si bien que le conduit de diffusion comporte un coude entre sa partie la plus proche du réservoir et sa partie terminale.

5. Chambre selon la revendication 4, **caractérisée en ce que** le coude du conduit de diffusion (27) est à l'intérieur de l'enveloppe.

6. Chambre selon la revendication 5, **caractérisée en ce que** le coude forme un angle compris entre environ 100 et 150°.

7. Chambre selon la revendication 2, **caractérisée en ce que** la partie du conduit de diffusion (43) la plus proche du réservoir (51) est sensiblement tangente à la paroi circulaire du réservoir.

8. Chambre selon la revendication 2, **caractérisé en ce que** l'enveloppe forme un dégagement (31) a un coin de son contour triangulaire, et la partie terminale du conduit de diffusion (27) débouche de l'enveloppe (17) à l'endroit du dégagement.

9. Chambre selon la revendication 2, **caractérisé en ce que** l'enveloppe a un orifice (35) qui traverse la paroi de base à proximité du côté du triangle opposé au sommet de forme effilée, l'orifice étant destiné au passage d'un fil de suture.

## Patentansprüche

1. Implantierbare Kammer zur Infusion eines Medikamentes, die dazu bestimmt ist, subkutan durch einen Schnitt in einen Körper implantiert zu werden, vom Typ mit:
- einem Speicher (15, 51, 69) für das Medikament,
- einer Zugangszone (26) zu dem Speicher, die am Scheitel desselben angeordnet, von außerhalb des Körpers zugängig und dazu bestimmt ist, das Füllen des Medikamentenspeichers zu erlauben, und
- einer Diffusionsleitung (27, 43, 63), die mit dem Speicher verbunden ist und sich nach außerhalb desselben erstreckt und so einen dem Speicher nahen Teil und einen äußeren Teil (27a) hat, und
- einer äußeren Hülle (17), welche den Speicher umgibt und eine Basiswand (19, 49) und eine äußere Seitenwand (21) besitzt, die von der Basiswand zu dem Scheitel des Behälters verläuft, wobei der Umriß der Basiswand (19, 49) im wesentlichen dreieckig ist, und derart, daß die Hülle (17) zu einem Scheitel des Dreiecks hin eine konisch zulaufende Form hat, und derart, daß:
- die Diffusionsleitung so beschaffen ist, daß ihr dem Speicher nächstliegender Teil in der Hülle eingehüllt ist und ihr äußerer Teil außerhalb der Hülle angeordnet ist und sich in im wesentlichen paralleler Richtung an derjenigen Seite des Dreiecks erstreckt, die dem Scheitel mit konisch zulaufender Form gegenüberliegt, **dadurch gekennzeichnet, daß** die Diffusionsleitung in einer Zone der Kammer angeordnet ist, die dem konisch zulaufenden Ende der Hülle gegenüberliegt.

2. Kammer nach Anspruch 1, **dadurch gekennzeichnet, daß** die Hülle (17) auch eine konisch zulaufende Form zum Scheitel des Dreiecks hin in der Richtung der Dicke senkrecht zu der Basiswand hat.

3. Kammer nach Anspruch 2, **dadurch gekennzeichnet, daß** der Speicher (15, 51, 69) einen in paralleler Richtung zu der Basiswand der Hülle im wesentlichen kreisrunden Querschnitt hat.

4. Kammer nach Anspruch 2, **dadurch gekennzeichnet, daß** der der Diffusionsleitung nächstliegende Teil des Speichers (15, 69) bezüglich der kreisrunden Wand des Speichers radial ist, so daß die Diffusionsleitung zwischen ihrem dem Speicher nächstliegenden Teil und ihrem Endteil eine Biegung aufweist.

5. Kammer nach Anspruch 4**, dadurch gekennzeichnet, daß** sich die Biegung der Diffusionsleitung (27) im Inneren der Hülle befindet.

6. Kammer nach Anspruch 5, **dadurch gekennzeichnet, daß** die Biegung einen Winkel zwischen etwa 100° und 150° bildet.

7. Kammer nach Anspruch 2, **dadurch gekennzeichnet, daß** der dem Speicher (51) nächstliegende Teil der Diffusionsleitung (43) im wesentlichen die kreisrunde Wand des Speichers tangiert.

8. Kammer nach Anspruch 2, **dadurch gekennzeichnet, daß** die Hülle einen Austritt (31) an einer Ecke ihrer dreieckigen Kontur bildet, und daß der Endteil der Diffusionsleitung (27) aus der Hülle (17) an der Stelle des Austritts herauskommt.

9. Kammer nach Anspruch 2, **dadurch gekennzeichnet, daß** die Hülle eine Öffnung (35) hat, welche die Basiswand in der Nähe derjenigen Seite des Dreiecks durchquert, welche dem Scheitel mit konisch zulaufender Form gegenüberliegt, wobei die Öffnung für den Durchgang eines Nahtfadens bestimmt ist.

## Claims

1. Implantable chamber for the infusion of a medicament, which chamber is to be implanted subcutaneously by way of an incision in a body, of the type which comprises:
- a medicament reservoir (15, 51, 69),
- a region (26) for access to the reservoir, which region is located at a vertex of the reservoir, is accessible from outside the body and is to enable the medicament reservoir to be filled, and
- a diffusion duct (27, 43, 63) which is connected to the reservoir and which extends outside the latter, the diffusion duct having an portion close to the reservoir and an external portion (27a), and
- an outer casing (17) surrounding the reservoir and having a base wall (19, 49) and an outer lateral wall (21) extending from the base wall to the top of the reservoir, the contour of the base wall (19, 49) being substantially triangular and such that the casing (17) has a shape tapered towards a vertex of the triangle, and such that:
- the diffusion duct is such that its portion closest to the reservoir is surrounded by the casing, and its external portion is located outside the casing and extends in a direction substantially parallel with the side of the triangle opposite the tapered vertex, **characterised in that** the diffusion duct is located in a region of the chamber opposite the tapered end of the casing.

2. Chamber according to claim 1, **characterised in that** the casing (17) also has a shape tapered towards the vertex of the triangle in the direction of the thickness perpendicular to the base wall.

3. Chamber according to claim 2, **characterised in that** the reservoir (15, 51, 69) has a substantially circular cross-section in the direction parallel with the base wall of the casing.

4. Chamber according to claim 2, **characterised in that** the portion of the diffusion duct closest to the reservoir (15, 69) is radial relative to the circular wall of the reservoir, so that the diffusion duct comprises a bend between its portion closest to the reservoir and its terminal portion.

5. Chamber according to claim 4, **characterised in that** the bend of the diffusion duct (27) is inside the casing.

6. Chamber according to claim 5, **characterised in that** the bend forms an angle of approximately from 100 to 150°.

7. Chamber according to claim 2, **characterised in that** the portion of the diffusion duct (43) closest to the reservoir (51) is substantially tangent to the circular wall of the reservoir.

8. Chamber according to claim 2, **characterised in that** the casing forms a recess (31) at a corner of its triangular contour, and the terminal portion of the diffusion duct (27) opens out from the casing (17) at the site of the recess.

9. Chamber according to claim 2, **characterised in that** the casing has an opening (35) which extends through the base wall in the vicinity of the side of the triangle opposite the tapered vertex, the opening being intended for the passage of a suture thread.
